# EUROPEAN PATENT SPECIFICATION

(11) **EP 0 512 051 B1**
(45) Date of publication and mention of the grant of the patent: **24.04.1996**
(21) Application number: 91903953.7
(22) Date of filing: 23.01.1991
(51) Int. Cl.: A61L 2/20

(54) **RESERVOIR DISPENSING SYSTEM FOR CHEMICAL STERILIZER**
TANKSPENDERSYSTEM FÜR CHEMISCHE STERILISATIONSVORRICHTUNG
SYSTEME DE DISTRIBUTION A RESERVOIR POUR STERILISATEUR CHIMIQUE

(30) Priority: 23.01.1990 US 468770
(43) Date of publication of application: 11.11.1992
(73) Proprietor: MDT CORPORATION, Rochester, NY 14623 (US)
(72) Inventor: SCHMOEGNER, John, C., Redondo Beach, CA 90277 (US); CRAMER, Michael, P., Victor, NY 14564 (US)
(74) Representative: Rambelli, Paolo
(86) International application number: US9100479
(87) International publication number: WO9111203

(56) References cited:
- US-A- 3 473 886
- US-A- 3 871 404
- US-A- 4 286 636
- US-A- 4 447 399
- US-A- 4 924 922

## Description

### BACKGROUND OF THE INVENTION

Field: This invention relates generally to sterilizers which generate chemical vapors. It is specifically directed to the chemical sterilant reservoirs associated with such sterilizers, and provides an improved structural arrangement for filling and sealing such reservoirs.

State of the Art: Chemical vapor sterilizers are well known. MDT Corporation of Torrance, California markets such devices under the trademark "CHEMICLAVE®," for example. Sterilizers of this type are characterized by a chamber into which a prescribed quantity of liquid sterilant chemical is introduced. The liquid is heated in the chamber, thereby creating chemical vapor sterilant. Modern systems include a reservoir for holding sufficient liquid sterilant chemical for several sterilization cycles. Sterilant is delivered in liquid form from the reservoir through a transport system to the sterilizing chamber.

From time to time, liquid is poured into the reservoir through a filling port to replenish the reserve supply. On occasion, an inappropriate substance, typically water, may be inadvertently poured into the reservoir. Subsequent operation of the sterilizer may then be dangerous, ineffective or otherwise detrimental.

Certain sterilizers are capable of operating in either a steam sterilization or chemical vapor sterilization mode. Such an apparatus is disclosed by U.S. Patent 4,447,399, the disclosure of which is incorporated by reference. Combination steam and chemical vapor sterilizers of this type include separate reservoirs and liquid transport systems for distilled water and liquid chemical sterilants, respectively. The opportunity thus exists for chemical sterilant to be inadvertently added to the water reservoir or for water to be inadvertently added to the chemical sterilant.

In recent years it has become an objective to avoid the discharge of chemical vapors to even a well-ventilated room. Because of pressure changes within a chemical vapor sterilizing system during its operation, there are instances in which the pressure in the liquid transport system can exceed the pressure above the liquid in the reservoir. Means must be provided to avoid the venting of chemical sterilant through the filling port of the reservoir. Conventionally, a plug is threaded into the filling port for this purpose. The plug must be removed to add chemical sterilant. Sometimes the plug becomes difficult or impossible to remove; sometimes it is discarded or lost.

There remains a need for a chemical sterilant reservoir arrangement which avoids the disadvantages of currently available such devices.

### SUMMARY OF THE INVENTION

According to the present invention, the filling port of a chemical sterilant reservoir is provided with a special valve. This valve is mutually adapted to a special nozzle associated with a container of liquid chemical sterilant. The nozzle also contains a valve. Both valves are normally closed so that liquid will not spill from the container, typically a bottle; neither will liquid or vapors escape from the reservoir in response to elevated pressure in the liquid transport system of the sterilizer. The two valves may be coupled to effect opening of both valves, thereby opening a liquid flow path from the container to the reservoir.

The normally closed reservoir valve blocks the passage of liquids poured into the filling port of the reservoir, thereby avoiding the inadvertent addition of unwanted liquids. Insertion of the nozzle of the storage container into the filling port operates both valves to permit the flow of liquid from the container into the reservoir. The nozzle may be inserted into a filling port lacking the necessary structure to open the nozzle valve, (such as the filling port of a distilled water reservoir), without effecting an inadvertent transfer of liquid from the container.

Structure within the reservoir filling port interacts with corresponding structures within the nozzle to effect the opening of both valves. Ideally, the reservoir valve opens prior to opening of the nozzle valve to assure that the liquid will not flow from the container unless the reservoir is open to receive it.

In its preferred embodiments, this invention constitutes an improvement to sterilisers of the type including a reservoir for liquid sterilant in which the reservoir has a filling port through which liquid is poured from a dispenser into the reservoir. Normally closed first valve means is operably associated with the filling port to isolate the interior of the reservoir from the atmosphere. This isolation prevents "blow back" venting through the reservoir. Normally closed second valve means is operably associated with the dispenser to prevent spillage of liquid from the dispenser. First structure carried by or otherwise mechanically linked to the first valve means is actuatable by applied force to open the first valve means. Similar second structure is mechanically linked to the second valve means. The first and second structures are arranged for coupling or mutual engagement, (usually in juxtaposition or abutment), so that each structure applies actuating force to the other. In this way, a liquid travel path is opened between the dispenser and the reservoir.

Ideally, the second valve means is at least partially insertable within the first valve means to bring the first and second structures into actuating engagement. The first structure preferably requires less actuating force than does the second structure so that the first valve means opens first when the second valve means is inserted into the first valve means. This sequence avoids spillage from the dispenser.

The first valve means is conveniently incorporated in the filling port with first structure actuatable in a longitudinal or axial direction with respect to the filling port. The second valve means is conveniently incorporated in the closure cap of the dispenser; e.g., as a nozzle valve. The second structure is also generally actuatable in a longitudinal or axial direction with respect to the dispenser. When the first and second structures are juxtaposed, the first and second structures are generally oriented on a common axis.

### BRIEF DESCRIPTION OF THE DRAWINGS

In the drawings, which illustrate what is currently regarded as the best mode for carrying out the invention,
FIG. 1 is a pictorial view of a reservoir tank and associated fixtures, including a filling funnel constituting a part of this invention;
FIG. 2 is a schematic cross-sectional view of the tank of FIG. 1 at the reference line 2-2 looking in the direction of the arrows in association with a portion of a dispenser container of this invention, also shown schematically in cross-section;
FIG. 3 is a schematic view similar to FIG. 2 with the container in a filling position with respect to the filling funnel;
FIG. 4 is similar to FIG. 3, but illustrates an alternative filling funnel structure;
FIG. 5 is an exploded view of the components of the filling funnel illustrated by FIGS. 2 and 3;
FIG. 6 is an exploded view of the components of the nozzle assembly illustrated by FIGS. 2, 3 and 4;
FIG. 7 is a pictorial view illustrating the valve assembly of FIG. 6 in assembled condition in association with a filling bottle.

### DESCRIPTION OF THE ILLUSTRATED EMBODIMENT

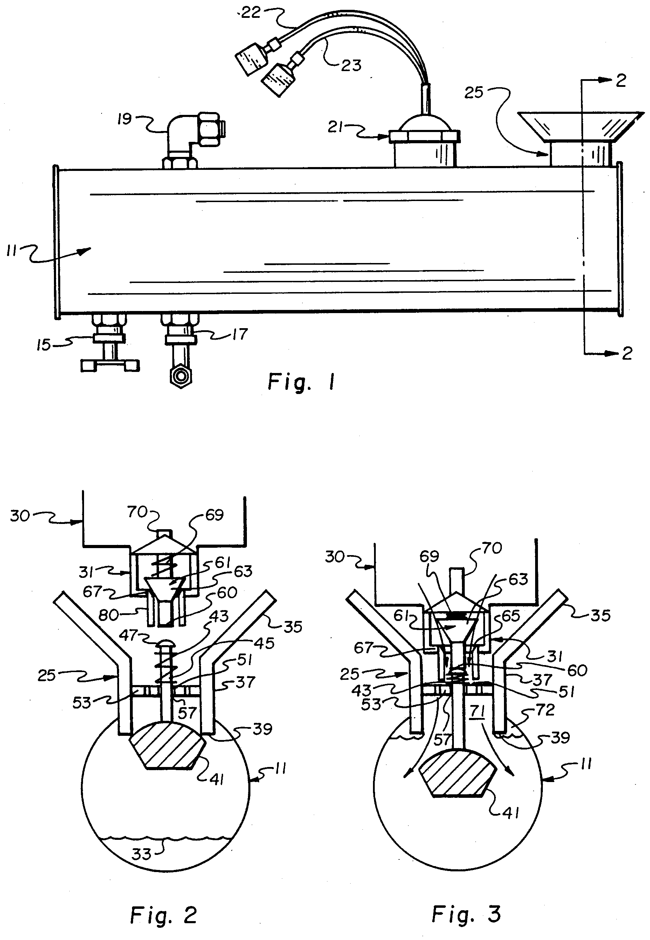

The reservoir tank 11 shown by FIG. 1 is conventional to the extent that it is sized appropriately to contain sufficient volume of liquid sterilant solution to provide the chemical vapors required for a convenient number of operating cycles of the sterilizer (not shown) of which it forms a part. The drain valve 15, plumbing fittings 17, 19 and level indicator 21 with its conductor leads 22, 23 are typically present in modern chemical vapor sterilizers. A filling funnel 25 is also typical, but the present invention utilizes a significantly modified such funnel 25 (see FIGS. 2, 3 and 5) in association with a dispenser container 30, having a closure cap 31 of special construction (see FIGS. 2, 3, 4, 6 and 7).

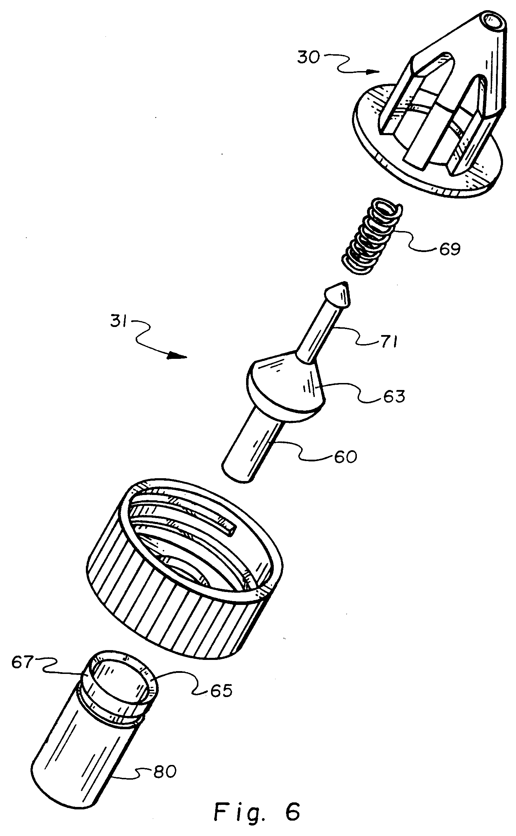

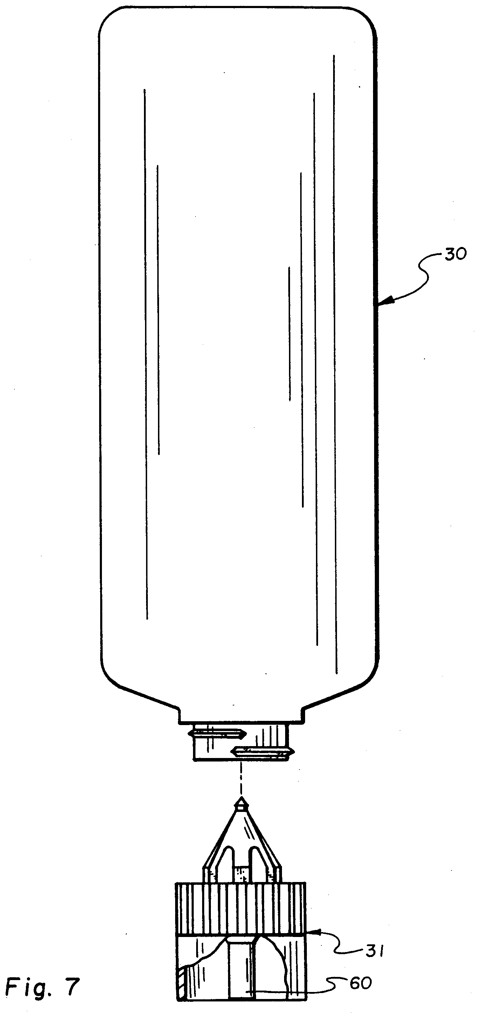

In the preferred embodiments, the level indicator 21 signals an associated device (not shown), such as a light, that the liquid level 33 in the tank 11 is sufficiently low that the tank 11 will receive the contents of the container 30. As illustrated by FIG. 7, a convenient such container 30 is a glass or plastic bottle of about 1/2 to about two liters capacity. The cap 31 is structured as a specialized nozzle (see FIG. 6).

FIGS. 2, 3 and 5 illustrate an improved filling funnel 25 for reservoir tank 11 containing potentially toxic or irritating liquids. The main body portion of the funnel 25 is formed as a hollow truncated cone 35 opening through a spout 37 in the fashion of a conventional funnel. The discharge end 39 of the spout 37 is normally sealed against fluid flow by a plug 41 which is biased into engagement with the end 39 by means of a compressed spring 43 (see FIG. 2). The spring 43 is positioned around a plunger 45 which connects to the plug 41 and functions as a spring follower as the spring 43 is further compressed (FIG. 3) between the head 47 of the plunger 45 and a static reaction surface 51 of an internal support member 53.

The plunger 45 reciprocates through the support member 53 through an appropriate opening 57 in response to pressure (or lack of pressure) applied by the depending end 60 of the stopper element 61 of the dispenser cap 31 to the plunger head 47. The stopper element 61 includes a frustoconical segment 63 biased into sealing engagement with a corresponding conical bore 65 (FIG. 3) in the cap closure plate 67 by a compressed spring 69. The spring 69 is retained by a follower extension 70 integral with the stopper element 61.

It is presently preferred to select springs 43 and 69 which assure reliable opening of both the funnel end 39 and the dispenser bore 65 to fluid flow. The dispenser spring 69 should ordinarily be stiffer than the funnel spring 43 (typically developing up to four or more items as much compression pressure) to assure that the solution will not flow from the dispenser 30 until the stopper 41 is dislodged, thereby permitting flow into the reservoir 11.

As shown, the funnel spout 37 projects into the interior of the reservoir 11. Solution will flow from the container 30 into the reservoir 11 until the passage 71 is full (FIG. 3), leaving an air gap 72 at the top of the reservoir. The volume of the air gap 72 may be adjusted by the depth of the distal end 39 of the funnel spout 37.

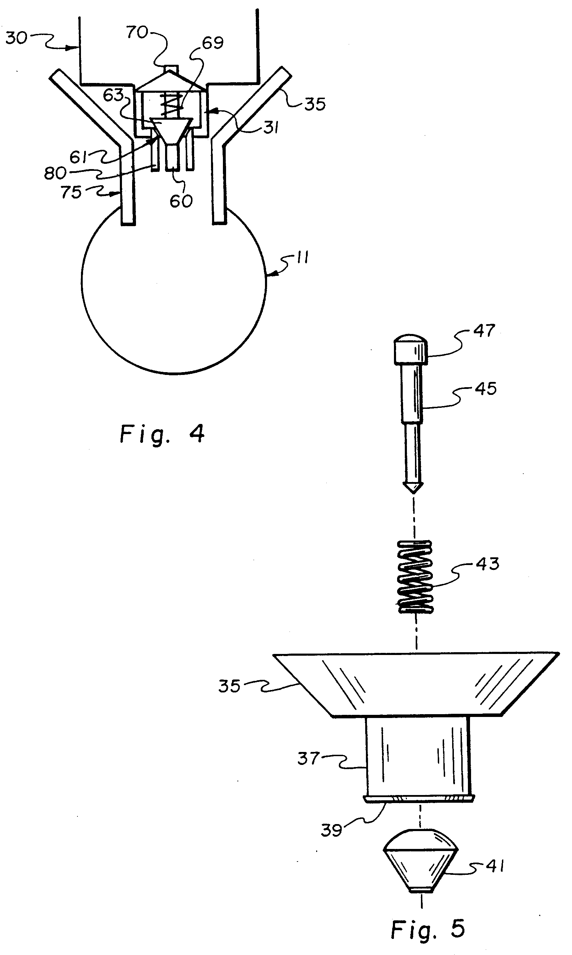

The structure of this invention can be combined in various ways to avoid dispensing chemicals into reservoirs intended to receive other chemicals or water. For example, sterilizers of the type disclosed by the aforementioned U.S. patent No. 4,447,399 are equipped with separate reservoirs which store water and chemical solution, respectively. It is important to avoid introducing liquid chemicals to the reservoir intended for storage of water. According to this invention, a filling funnel 75 such as that illustrated by FIG. 4 is provided for the water reservoir, while a funnel of the type illustrated by FIGS. 2 and 3 is provided for chemical sterilant reservoir. Insertion of the nozzle 31 into the funnel 75 is ineffective to open the conical bore 65. Thus, the consistent use of filling bottles 30 with specialized caps 31 of the type illustrated by FIGS. 6 and 7 for the storage and dispensing of liquid chemical sterilant assures against inadvertent contamination of the steam circuit of the system. Equally important, the normally closed funnels 25 prevent the flow of liquid beyond the plug 41 if those liquids are dispensed from containers which lack the structural features required to dislodge the plug 41.

The coordinated valve system illustrated avoids the potentially hazardous open flow of liquids from a container to the filling funnel 25. It also avoids the need for a removable plug to isolate the reservoir from the atmosphere. Preferably, a nozzle extension 80 is provided to isolate the depending plunger end 60 from sources of inadvertent applied force.

Reference herein to specific details of the illustrated embodiments is not intended to restrict the scope of the appended claims.

## Claims

1. A sterilizer comprising:
- a reservoir for liquid chemical sterilant, said reservoir including a filling port through which liquid is poured from a dispenser into said reservoir;
- normally closed first valve means operably associated with said filling port to isolate the interior of said reservoir from the atmosphere;
- normally closed second valve means operably associated with said dispenser to prevent spillage of liquid from said dispenser;
- first structure associated with said first valve means actuatable by applied force to open said first valve means; and
- second structure associated with said second valve means actuatable by applied force to open said second valve means;
- said first and second structure being arranged for mutual engagement whereby each applies actuating force to the other, thereby opening a liquid travel path between said dispenser and said reservoir.

2. The sterilizer according to claim 1, wherein said second valve means is insertable within said first valve means, to bring said first and second structures into engagement.

3. The sterilizer according to claim 2, wherein said first structure is constructed and arranged to be actuatable with less force than is said second structure so that said first valve means opens first when said second valve means is inserted into said first valve means.

4. A sterilizer according to Claim 3 wherein said first and second structures are constructed to be actuated against the force of respective first and second springs, and the first said spring is weaker than said second spring.

5. A sterilizer comprising:
a first reservoir for liquid chemical sterilant; a second reservoir for water; a first filling port through which liquid chemical sterilant is poured from a dispenser into said first reservoir; and a second filling port through which water is introduced to said second reservoir;
normally closed first valve means disposed within said first filling port to isolate the interior of said first reservoir from the atmosphere;
normally closed second valve means disposed within a cap for said dispenser to prevent spillage of liquid from said dispenser, said cap including a nozzle extension constructed and arranged to enter said first filling port, thereby to bring said first valve means into contact with said second valve means;
said first and second valve means being mutually constructed so that upon forcible contact with each other, both said first and second valve means open, thereby opening a liquid travel path between said dispenser and said first reservoir.

6. The sterilizer according to Claim 5 wherein said second filling port is constructed and arranged to permit full insertion of said nozzle extension without opening said second valve means.

7. The sterilizer according to Claim 5 including:
first structure mechanically linked to said first valve means actuatable by applied force to open said first valve means;
second structure mechanically linked to said second valve means actuatable by applied force to open said second valve means;
said first and second structures being arranged for mutual engagement so that each applies actuating force to the other.

8. The sterilizer according to Claim 7 wherein said second structure is contained within said nozzle extension.

9. The sterilizer according to Claim 7 wherein said first structure is constructed and arranged to be actuatable with less force than is said second structure so that said first valve means opens first when said nozzle extension is inserted into said first filling port.

10. The sterilizer according to Claim 9 wherein said first and second structures are constructed to be actuated against the force of respective first and second springs, and the first said spring is weaker than said second spring.

## Patentansprüche

1. Sterilisator mit:
- einem Reservoir für flüssiges chemisches Sterilisiermittel, wobei das Reservoir eine Füllöffnung einschließt, durch welche Flüssigkeit aus einem Spender in das Reservoir eingegossen wird;
- normalerweise geschlossenen ersten Ventilmitteln, die mit der Füllöffnung verbunden sind um das Innere des Reservoirs von der Atmosphäre zu isolieren;
- normalerweise geschlossenen zweiten Ventilmitteln, die mit dem Spender verbunden sind um das Vergießen von Flüssigkeit aus dem Spender zu verhindern;
- einer ersten Struktur, die mit den ersten Ventilmitteln verbunden ist und durch das Aufbringen einer Kraft zum Öffnen der ersten Ventilmittel betätigbar ist; und
- einer zweiten Struktur, die mit den zweiten Ventilmitteln verbunden ist und durch Aufbringen einer Kraft zum Öffnen der zweiten Ventilmittel betätigbar ist;
- wobei die erste und die zweite Struktur zum gegenseitigen Eingriff angeordnet sind, wodurch jede eine Betätigungskraft auf die andere aufbringt und dadurch einen Fließpfad für die Flüssigkeit zwischen dem Spender und dem Reservoir öffnet.

2. Sterilisator nach Anspruch 1, bei dem die zweiten Ventilmittel in die ersten Ventilmittel einsetzbar sind, um die ersten und zweiten Strukturen miteinander in Eingriff zu bringen.

3. Sterilisator nach Anspruch 2, bei dem die erste Struktur aufgebaut und angeordnet ist, um durch eine kleinere Kraft betätigbar zu sein als die zweite Struktur, so daß die ersten Ventilmittel sich zuerst öffnen, wenn die zweiten Ventilmittel in die ersten Ventilmittel eingesetzt werden.

4. Sterilisator nach Anspruch 3, bei dem die ersten und zweiten Strukturen aufgebaut sind, um gegen die Kraft von entsprechenden ersten und zweiten Federn betätigt zu werden und die erste Feder schwächer als die zweite Feder ist.

5. Sterilisator mit:
einem ersten Reservoir für flüssiges chemisches Sterilisiermittel; einem zweiten Reservoir für Wasser; einer ersten Füllöffnung, durch welche flüssiges chemisches Sterilisiermittel aus einem Spender in das erste Reservoir gefüllt wird; und einer zweiten Füllöffnung, durch welche Wasser in das zweite Reservoir eingeführt wird;
normalerweise geschlossenen ersten Ventilmitteln, die in der ersten Füllöffnung angeordnet sind um das Innere des ersten Reservoirs von der Atmosphäre zu isolieren;
normalerweise geschlossenen zweiten Ventilmitteln, die in einer Kappe für den Spender angeordnet sind, um das Vergießen von Flüssigkeit aus dem Spender zu verhindern, wobei die Kappe eine Düsenverlängerung einschließt, welche aufgebaut und angeordnet ist, um in die erste Füllöffnung einzutreten und dadurch die ersten Ventilmittel mit den zweiten Ventilmitteln in Kontakt zu bringen;
wobei die ersten und zweiten Ventilmittel beide derart konstruiert sind, daß sie sich bei Kontakt miteinander unter Krafteinwirkung beide öffnen und dadurch einen Fließpfad für die Flüssigkeit zwischen dem Spender und dem ersten Reservoir öffnen.

6. Sterilisator nach Anspruch 5, bei dem die erste Füllöffnung aufgebaut und angeordnet ist, um das vollständige Einsetzen der Düsenverlängerung zuzulassen, ohne die zweiten Ventilmittel zu öffnen.

7. Sterilisator nach Anspruch 5 einschließend:
eine erste Struktur, die mechanisch mit dem ersten Ventilmittel verbunden ist und durch eine aufgebrachte Kraft betätigbar ist, um die ersten Ventilmittel zu öffnen;
eine zweite Struktur, die mechanisch mit dem zweiten Ventilmittel verbunden und durch eine aufgebrachte Kraft betätigbar ist, um die zweiten Ventilmittel zu öffnen;
wobei die ersten und zweiten Strukturen zum gegenseitigen Eingreifen angeordnet sind, so daß jede eine Betätigungskraft auf die andere ausübt.

8. Sterilisator nach Anspruch 7, bei dem die zweite Struktur in der Düsenverlängerung aufgenommen ist.

9. Sterilisator nach Anspruch 7, bei dem die erste Struktur aufgebaut und angeordnet ist, um durch eine kleinere Kraft betätigbar zu sein, als es die zweite Struktur ist, so daß die ersten Ventilmittel sich zuerst öffnen, wenn die Düsenverlängerung in die erste Füllöffnung eingesetzt wird.

10. Sterilisator nach Anspruch 9, bei dem die ersten und zweiten Strukturen aufgebaut sind, um gegen die Kraft von entsprechenden ersten und zweiten Federn betätigt zu werden, und die erste Feder schwächer als die zweite Feder ist.

## Revendications

1. Stérilisateur, comprenant :
- un réservoir d'un produit chimique liquide de stérilisation, le réservoir contenant un canal de remplissage par lequel un liquide est versé d'un distributeur dans le réservoir,
- une première soupape normalement fermée associée au canal de remplissage afin qu'elle isole l'intérieur du réservoir de l'atmosphère,
- une seconde soupape normalement fermée associée au distributeur et destinée à éviter la dispersion du liquide du distributeur,
- une première structure associée à la première soupape et destinée à être commandée par une force appliquée pour l'ouverture de la première soupape, et
- une seconde structure associée à la seconde soupape et destinée à être commandée par la force appliquée pour l'ouverture de la seconde soupape,
- la première et la seconde structure étant destinées à être en coopération mutuelle de manière que chacune d'elles applique une force de manoeuvre à l'autre et provoque ainsi l'ouverture d'un trajet de passage de liquide entre le distributeur et le réservoir.

2. Stérilisateur selon la revendication 1, dans lequel la seconde soupape est destinée à pénétrer dans la première soupape pour mettre en contact la première et la seconde structure.

3. Stérilisateur selon la revendication 2, dans lequel la première structure a une construction et une disposition telles qu'elle peut être manoeuvrée avec une force inférieure à celle de la seconde structure afin que la première soupape s'ouvre d'abord lorsque la seconde soupape est introduite dans la première soupape.

4. Stérilisateur selon la revendication 3, dans lequel la première et la seconde structure ont une construction telle qu'elles sont destinées à être manoeuvrées malgré la force antagoniste d'un premier et d'un second ressort respectifs, le premier ressort étant moins puissant que le second.

5. Stérilisateur, comprenant :
un premier réservoir d'un produit chimique liquide de stérilisation, un second réservoir d'eau, un premier canal de remplissage par lequel le produit chimique liquide de stérilisation est versé d'un distributeur dans le premier réservoir, et un second canal de remplissage par lequel de l'eau est introduite dans le second réservoir,
une première soupape normalement fermée placée dans le premier canal de remplissage et destinée à isoler l'intérieur du premier réservoir de l'atmosphère,
une seconde soupape normalement fermée placée dans un capuchon du distributeur et destinée à éviter la dispersion du liquide du distributeur, ce capuchon comprenant un prolongement de buse dont la construction et la disposition sont telles qu'il peut pénétrer dans le premier canal de remplissage et mettre ainsi la première soupape au contact de la seconde soupape,
la première et la seconde soupape ayant des constructions mutuelles telles que, lors d'un contact à force l'une avec l'autre, la première et la seconde soupape s'ouvrent et ouvrent ainsi un trajet de passage du liquide entre le distributeur et le premier réservoir.

6. Stérilisateur selon la revendication 5, dans lequel le second canal de remplissage a une construction et une disposition qui permettent l'introduction complète du prolongement de la buse sans ouverture de la seconde soupape.

7. Stérilisateur selon la revendication 5, comprenant :
une première structure liée mécaniquement à la première soupape et destinée à être commandée par une force appliquée pour l'ouverture de la première soupape,
une seconde structure liée mécaniquement à la seconde soupape et destinée à être commandée par la force appliquée pour l'ouverture de la seconde soupape,
la première et la seconde structure étant disposées afin qu'elles coopèrent mutuellement et que chacune applique une force de manoeuvre à l'autre.

8. Stérilisateur selon la revendication 7, dans lequel la seconde structure est logée à l'intérieur du prolongement de la buse.

9. Stérilisateur selon la revendication 7, dans lequel la première structure a une construction et une disposition telles qu'elle peut être manoeuvrée avec une force plus faible que la seconde structure si bien que la première soupape s'ouvre en premier lorsque le prolongement de la buse est introduit dans le premier canal de remplissage.

10. Stérilisateur selon la revendication 9, dans lequel la première et la seconde structure ont une construction telle qu'elles peuvent être manoeuvrées malgré la force antagoniste d'un premier et d'un second ressort respectif, et le premier ressort est moins puissant que le second.
